# FASCICULE DE BREVET EUROPEEN

(11) **EP 3 789 048 B1**
(45) Date de publication et mention de la délivrance du brevet: **07.06.2023**
(21) Numéro de dépôt: 19020506.2
(22) Date de dépôt: 03.09.2019
(51) Int. Cl.: A61L 27/48, A61L 27/56

(54) **SCAFFOLD MÉNISCAL COMPOSITE COMPRENANT DU COLLAGÈNE HUMAIN RECOMBINANT**
KOMPOSITGERÜST ENTHALTEND EIN HUMANES REKOMBINATES KOLLAGENS ZUR WIEDERHERSTELLUNG DES MENISKUS
MENISCAL COMPOSITE SCAFFOLD COMPRISING HUMAN RECOMBINANT COLLAGEN

(43) Date de publication de la demande: 10.03.2021
(73) Titulaire: Assor, Michel, 13009 Marseille (FR)
(72) Inventeur: Assor, Michel, 13009 Marseille (FR)
(74) Mandataire: Roman, Alexis

(56) Documents cités:
- WO-A1-2013/118125
- WO-A1-2015/134028
- US-A1- 2007 186 312

## Description

RESUME : L'étendue de la protection est définie par les revendications. Dans un premier aspect, l'invention a pour objet un échafaudage composite selon les revendications 1 à 8 et un implant méniscal selon les revendications 9 à 10. Dans un deuxième aspect, l'invention a pour objet un procédé de fabrication de l'échafaudage composite selon les revendications 11 à 13.

La présente invention concerne un Implant ou scaffold méniscal appelé Collafit, composite en mousse poreuse à base de deux structures : 1.Structure mécanique de polymères. de polyuréthane de Polycaprolactone PCL, et de deux polymères Poly-lactic-co-glycolic acid PLGA, et polycarbanate uréthane PCA, avec éventuellement un hydrogel physique supramoléculaire ; 2. Associée à une structure biologique de collagène type I ou II recombinant humain de plante, non animal ; Implant scaffold destiné à remplacer le ménisque médiale ou latéral gravement endommagé du genou, avec fibres renforçant sa résistance en particulier périphérique, dégradable et colonisable par du tissus fibrocartilage, permettant la protection du cartilage des dégradations arthrosiques consécutives à la perte méniscale. Mais aussi destiné à être implanté dans l'arthrose terminale grade 3 ou 4 du genou, pour régénération de cartilage, en association avec d'autres gestes de traitement des désordres mécaniques (déformation axiale, instabilité ligamentaire, ...) et la thérapie cellulaire (cellules souches mésenchymateuses Csm, et plasma riche en plaquettes Prp.

CONTEXTE : Les ménisques, médial et latéral, sont des joints fibrocartilage placés entre condyle et tibia, en forme de C et triangulaire en forme de coin en coupe transversale. Ils sont essentiels dans la transmission et répartition des charges (compression, traction). Les lésions méniscales traumatiques ou arthrosiques sont très fréquentes, et du fait de leur faible vascularisation, la cicatrisation spontanée est faible, et une intervention chirurgicale par méniscectomie arthroscopique plus ou moins partielle est le plus souvent nécessaire, malgré l'importance du ménisque, aux résultats cliniques médiocres. Il a été démontré une corrélation directe entre le capital méniscal restant et les lésions du cartilage articulaire (1, 2). Actuellement, il n'y a pas de traitement efficace à long terme pour remplacer une perte importante (plus de 50%) du ménisque. Le traitement par allogreffe de ménisque n'est pas fiable à long terme, avec infiltration cellulaire limitée, disponibilité limitée, problème de préservation et de transmission de maladie. Les progrès de l'ingénierie tissulaire ont abouti la réalisation de scaffold acellulaire en utilisation clinique, permettant la colonisation tissulaire. Les deux premiers implants scaffold méniscal poreux et permettant la croissance cellulaire, et d'utilisation clinique étaient : le ménisque à base de collagène de tendon d'Achille bovin Ménaflex ou CMI (Collagen méniscal implant, Société Stryker) (3) ; et le ménisque à base de mousse de polyuréthane Actifit (4). Notre équipe a une importante activité de pose de scaffold de ménisque dans l'arthrose grade 4 du genou avec thérapie cellulaire et traitement des lésions mécaniques du genou, avec plusieurs centaines de pose depuis 2012, dans cette indication d'arthrose terminale chez le sujet encore jeune pour éviter la prothèse de genou. L'implant Actifit a été utilisé, avec communication des résultats à la SFA de 2017 (14) ; puis au retrait du marché de Actifit pour problème financier en mars 2017, nous avons utilisé l'implant CMI dès avril 2017.

Cependant, ces scaffolds, surtout pour le CMI collagène, sont fragiles, se déchirant facilement, avec résistance faible à la pression et à la traction, et nécessitant un mur méniscal intact, pour résister aux forces de traction périphérique. Les études rapportent des résultats contradictoires, avec présence inconstantes de tissus fibrocartilage, colonisation incomplète, implants extrudés, et surtout pour le ménisque collagène CMI, implant collabé, partiellement ou totalement résorbé, poursuite du processus arthrosique, et problème de sepsis post-opératoire, de sous-produit cytotoxiques liés à la réticulation ; ainsi qu'une allergie dans 0,5% des cas aux protéines animales responsables de destruction inflammatoire de cartilage.

Ces deux produits ont une structure amorphe non appropriée pour un remplacement du ménisque. Le ménisque Actifit est depuis avril 2017, retiré du marché pour faillite. Le scaffold collagène de tendon d'Achille Bovin Ménaflex CMI labo Stryker, en particulier, est un implant inadapté à son indication de remplacement du ménisque : sa fragilité est telle, que lors de sa fixation intra articulaire par un système de type Fastfix de Smith and Nephew, ou Air de Stryker, la poussée de l'aiguille de pose de la suture fait collaber et plicaturer l'implant, qui est repoussé dans les tissus en dehors du plateau tibial (sur lequel il a été positionné) ; son perçage est délicat, nécessitent des mouvements de rotations permanents, et non une pression directe qui luxe l'implant hors du plateau tibial ; alors que l'implant en Polyuréthane Actifit était facile à fixer. Souvent, lors des premières sutures de sa partie postérieure, du fait de sa fragilité et légèreté, et son absence de rugosité, l'implant se positionnait de façon verticale et non plane parallèle au plateau tibial ; et il se plicaturait fréquemment. Sa texture inadaptée rendait sa pose et sa fixation difficiles.

Les stratégies actuelles d'ingénierie tissulaire utilisaient des matériaux biologiques tels que le collagène (5), l'hyaluronane (6), et la soie (7), ainsi que des polymères synthétiques tels que l'acide polylactique (8), l'acide polyglycolique (9), la polycaprolactone (10,11) et le polyéthylène, alcool polyvinylique (12). Plusieurs études à court terme ont montré une résistance à la compression, la morphologie des cellules fibrochondrocytaires et l'expression du collagène, mais la plupart ont échoué à plus long terme en raison de la rupture de l'implant et / ou de la détérioration de l'articulation (13).

Il existe donc un besoin d'un scaffold biologique, biodégradable et avec revascularisation et colonisable par du tissu fibrocartilage, et biocompatible sans dégradation de produit toxique, ayant les propriétés mécaniques du ménisque pour le traitement des graves lésions méniscales. Ce scaffold doit avoir une plus grande résistance à la compression normale condyle-tibia (pour éviter le collapsus des pores et la déchirure), à la traction (éviter la déchirure), et une plus grande résistance à la traction périphérique (éviter la déchirure et l'extrusion). Intervention difficile, il doit être facilement posé, en évitant les sutures trans- osseuses tibiales alourdissant inutilement le geste opératoire, et se démocratiser dans la communauté orthopédique. Les ménisques de remplacement actuels sont fragiles, se résorbent et se détruisent fréquemment notamment pour le CMI-Ménaflex collagène de tendon bovin de Stryker ; et s'avèrent limités concernant la colonisation souvent incomplètes ou absente de tissus fibro-cartilages dans les pores du scaffold ; et concernant la répartition des pressions entre fémur et tibia. Le ménisque de remplacement doit supporter la pression normale fémur-tibia, et doit être renforcée en périphérie externe pour supporter les contraintes en traction. Si les recherches et les tests sur le ménisque de remplacement sont toujours en cours, et notamment ceux récents utilisant des matériaux non résorbables comme le Kevlar, polyéthylène, Persaud (17) ; Elsner (18) ; avec un risque certain d'usure et qui n'a pas de place dans l'esprit d'un implant de remplacement du ménisque et de le transformer en fibrocartilage, aucune solution viable n'est proposée.

WO 2013/118125 A1 décrit un matériau composite comprenant des fibres de collagènes dans une matrice de polyuréthane. D1 décrit aussi l'utilisation de polymères biodégradables tels que le PLGA comme matrices. Ces matériaux composites peuvent être utilisés comme implant méniscal.

INTERÊT DE L'INVENTION. Scaffold de ménisque dans le traitement non prothétique de l'arthrose grave du genou par régénération de cartilage. La présente invention concerne un implant ou scaffold méniscal composite, mousse poreuse, appelé COLLAFIT, marque revendiquée, composé de : 1. polyuréthane de haut poids moléculaire à base de poly- ε- caprolactone (PCL), 1,4- butanédiol (BDO) et diisocyanate, associé à d'autres polymères: Poly-lactic-co-glycolic acid PLGA, et Polycarbanate uréthane PCA ; préparation par procédé de polymérisation en plusieurs étapes ; éventuellement un hydrogel physique ; 2. et de collagène humain type I ou II recombinant non animal issu de plante, principalement de tabac, par ingénierie tissulaire ; biocompatible, biodégradable, et à régénération et colonisation tissulaire ; et remplaçant la perte, - traumatique, chirurgicale ou arthrosique - du ménisque du genou, pour prévenir ou traiter l'arthrose du genou, par régénération de cartilage, induite par les gestes de régénération des defects de cartilage (microforages et thérapie cellulaire) et les corrections préalables ou simultanées des défauts mécaniques de lutte contre l'hyperpression des zones d'arthrose : ostéotomie si désaxation, reconstruction ligamentaire si instabilité, et libération section allongement systématique sous arthroscopie du ligament collatéral médial ou latéral selon le siège de l'arthrose (14), et de décompression de l'arthrose.

La régénération de cartilage dans l'arthrose grade 4 du genou et la possibilité d'éviter la prothèse de genou chez le patient encore jeune et performant, a été prouvée lors d'un essai clinique autorisée par l'ANSM et CPP (France) de 2010 à 2014, d'utilisation en un seul temps de cellules souches mésenchymateuses Csm de moelle osseuse aspirée concentrée, en réalisant sous arthroscopie des micrforages des défects de cartilage condyle-tibia, en libérant le ligament collatéral médial ou latéral ; et respectant les critères d'inclusion : bon axe ou déformation corrigée par ostéotomie au préalable ou simultanément à l'arthroscopie; stabilité ligamentaire ou instabilité traitée par reconstruction, au préalable ou simultanément ; et présence d'au moins 2/3 du ménisque. Les résultats ont été prouvées lors des communications scientifiques aux congrès Sofcot de 2013 et 2014 (15,16).

La perte méniscale est fréquente, responsable d'arthrose du genou : problème de santé publique touchant près de 40% de la population mondiale, l'arthrose grave du genou de grade 4 est fréquent chez le sujet jeune avec perte méniscale. Le traitement par implantation sous arthroscopie du genou de la greffe de ménisque dans l'arthrose terminale grade 4/4, couplé au traitement de l'arthrose par microforages des zones arthrosiques avec thérapie cellulaire (plaquettes concentrées plasma riche en plaquettes PRP, ou cellules souches de moelle osseuse concentrée, selon taille de l'arthrose si plus de 6 cm2), et après avoir traité les lésions mécaniques de désaxation par ostéotomie, et d'instabilité ligamentaire par reconstruction ligament ; et libération décompression systématique sous arthroscopie du ligament collatéral médial ou latéral, permet chez le patient jeune à bonne performance physique, d'éviter la prothèse de genou.

La communication scientifique de décembre 2017 à la Société Francophone d'Arthroscopie (SFA) (14) sur les résultats de l'implant méniscal polyuréthane dans l'arthrose grade 4 du genou, confirme les bons résultats sur la régénération de cartilage ; confirme les résultats insuffisant de la thérapie cellulaire et du traitement des lésions mécaniques du genou à arthrose grade 4 si la perte de ménisque de plus de 50% n'est pas remplacée par un implant méniscal.

La communication scientifique SFA de décembre 2017 confirme l'importance primordial, pour la mise en place sous arthroscopie de l'implant méniscal, de la libération relâchement de la capsule et du ligament collatéral médial ou latéral rétractés par la perte de le hauteur de cartilage. Leur section allongement permet en plus de décomprimer le compartiment arthrosique, de réduire le pincement complet de l'arthrose grade 4 en l'ouvrant suffisamment pour permettre la régénération de cartilage, qui reste souvent au contact os-os malgré l'ostéotomie éventuelle. Et surtout elle facilite grandement la mise en place du scaffold méniscal, son placement et sa fixation par multiples sutures.

### RESUME DESCRIPTION DE L'INVENTION

L'implant ou scaffold de remplacement du ménisque est en matériau composite biodégradable aux produits de dégradation non toxiques, matériau en mousse poreuse, composé de deux structures mécanique et biologie : (1). une structure à bonne tenue mécanique mousse et poreux en polyuréthane à base de polycaprolactone, et divers polymères incorporés incluant le Poly-lactic-co-glycolic acid PLGA, et le Polycarbanate uréthane PCA ; éventuellement un hydrogel physique supra moléculaire ; (2). et d'une structure biologique à base de collagène humain de type I ou II recombinant tiré de plante de tabac (ou autres plantes), non animal, proche du collagène humain, et facteur de stimulation de la colonisation vasculaire et cellulaire fibrocartilage de l'implant.

Des accords de partenariat de fabrication ont été signés avec un laboratoire de polymères et des laboratoires de biotechnologie pour le collagène humain recombinant type I ou II à base de plante

Selon un autre aspect, la présente invention propose une structure mécanique polyuréthane de haut poids moléculaire et polymère, résorbables, naturel ou synthétique, à plus haute tenue mécanique que les implants de l'art antérieur, résistant à la déchirure et à la traction, mais aussi à la pression fémorale exerçant une traction périphérique, utilisant l'implant polyuréthane Actifit, (société Orteq) décrit dans WO2015134028(A1) qui a reçu un agrément européen d'utilisation chez l'homme; et renforcé par la présence d'autres polymères PLGA et PCA ; et une structure biologique de collagène recombinant de plante.

Dans un autre mode de réalisation, la structure mécanique du scaffold est une combinaison de polymères résorbables. On fait réagir le poly- ε- caprolactone (PCL) avec le 1,4 butanédiol, formant un macrodiol ; et que l'on fait réagir avec un diisocyanate, pour obtenir un macrodiisocyanate ; puis réaction par procédé de polymérisation en plusieurs étapes avec PLGA et PCA.

Dans un autre mode de réalisation : le Polylactide-co-glycolide acide PLGA est utilisé dans une proportion de 50-50% pour le lactide et glycolide, avec une meilleur réponse biologique selon l'étude de Peng et al en 2018 (19). Dans un autre mode de réalisation, du polycarbonate uréthane PCA est utilisé (20), en réalisant des bâtonnets de PCA de renforcement de sa périphérie externe pour la résistance à la traction et pour la fiabilité des sutures de l'implant sur le mur méniscal restant ou sur la capsule si absence de mur méniscal. Le PCA a été largement testé dans l'industrie médicale et approuvé par la FDA, en raison de ses excellentes propriétés mécaniques, de sa biostabilité et de sa biocompatibilité. Le PCA a une grande capacité de résistance et de lubrification en raison de leur nature hydrophile, ainsi des propriétés de faible frottement essentielles au remplacement méniscal. Le PCA est selon un autre mode de fabrication mélangé à des fibres de polyéthylène PE, pour une plus grande rigidité. Le PCA seul ne donnerait pas de résultats satisfaisants selon une étude animale (21), avec poursuite de la dégradation du cartilage fémoro-tibial. Un hydrogel physique supramoléculaire peut être associé.

Selon un autre mode de réalisation, le PCA peut être fabriqué via une méthode de polymérisation en solution en 2 étapes, utilisant entres autres, héxanédiol, diisocyanate, butanédiol. L'homme de l'art saura utiliser les réactifs pour obtenir le matériau à résistance proche du tissu méniscal.

Dans un autre mode de réalisation, des tests mécaniques de résistance à la traction, à la pression, à la déchirure transversale, à l'aide d'une machine d'essai de matériaux type Zwick Roell.

Selon un autre mode de réalisation, les propriétés mécaniques du matériaux composite polymères et collagène recombinant de plante peuvent être personnalisées et se rapprocher de celles du ménisque humain, ce qui n'est pas le cas des implants d'utilisation clinique actuels (polyuréthane Actifit, et Collagène bovin Ménaflex CMI Stryker), peu résistant et colonisable, avec une fragilité particulière pour le collagène bovin CMI qui se déchire comme un buvard mouillé dans un milieu liquide.

Le collagène recombinant humain RH collagène de plantes (tabac). Utilisation dans le scaffold composite de l'invention, du collagène humain type I ou II recombinant de plante de tabac, structure biologique associée à la structure mécanique de polymères décrite ciavant, par fusion ou incorporation chimique, cross linking et lyophilisation ; ou impression 3D.

Dans un mode de réalisation, l'objet de l'invention est un échafaudage méniscal de remplacement du ménisque, en composite mousse poreuse de polyuréthane et de polymères PLGA et PCA, selon la réalisation décrite ci-dessus, avec une forme unique pour le remplacement du ménisque interne et/ou externe ; mélangé par cross-linking ou par impression 3D sur l'implant polymères, à des couches de fibres de collagène humain recombinant sur plante, non animal ; collagène type I ou II provenant de plants de tabacs génétiquement modifiés, ou Rh Collagène. Il est similaire au collagène humain, sans prions, ni de germe pathogène, ni allergène, ni réponse immunologique.. Il est similaire au collagène humain, sans prions, ni de germe pathogène, ni allergène, ni réponse immunologique. Le collagène de type I (gènes COL 1A1 et COL 1A2) est présent dans la peau, l'os, les tendons ; et le collagène de type Il (gènes COL 2A1) est présent dans le cartilage hyalin.

Le laboratoire Collplant, (Israël), réalise le collagène recombinant humain à partir de plants de tabac, de type I.

D'autres laboratoires utilisent le même type de réalisation de collagène humain recombinant de plantes et de type II.

Tous les types de collagène recombinant humain d'autres végétaux.

D'autres sociétés de biotechnologie avec expertises sur les protéines recombinantes, et l'ingénierie de plantes, avec laboratoires utilisant des serres, peuvent être associées.

La production de RhCollagen (collagène humain recombinant) commence lorsque des semis de plants de tabac génétiquement modifiés avec des gènes humains codant pour la production de collagène sont distribués dans des serres qualifiées, où ils sont cultivés jusqu'à maturité, ce qui prend environ huit semaines. Les feuilles de tabac sont ensuite récoltées et transformées en un extrait qui subit une purification jusqu'à la production du produit final de rhCollagène. Le produit résultant, le collagène humain vierge, sont les caractéristiques les plus importantes de la production végétale.

Le RhCollagène a démontré une fonction biologique supérieure par rapport à tout collagène dérivé de tissu, que ce soit à partir de tissus animaux ou humains selon les données publiées dans des publications scientifiques examinées par des pairs. Le RhCollagen peut être fabriqué sous différentes formes, formes et viscosités, y compris les gels, pâtes, éponges, feuilles, membranes, fibres et couches minces, qui ont tous été testés in vitro et sur des modèles animaux et prouvés supérieurs aux produits dérivés de tissus.

En raison de son homogénéité, rhCollagen peut produire des fibres et des membranes de haut ordre moléculaire, ce qui signifie que toutes les molécules sont orientées dans le même sens, ce qui permet la formation de produits de réparation tissulaire avec des propriétés physiques distinctives, notamment une meilleure résistance à la traction due à l'alignement des fibres de collagène, de transparence, et la capacité à atteindre des concentrations élevées de collagène à de faibles viscosités

Le collagène humain recombinant de type I ou II, rhCollagen I ou II, est identique au collagène produit par le corps humain. Le type I est présent dans l'os, les tendons et la peau ; le type II est présent dans le cartilage et c'est le type recherché dans l'inclusion de l'implant méniscal.

Les principaux avantages des produits utilisant le rhCollagène, par rapport à ceux utilisant du collagène dérivé d'animaux ou de tissus cadavériques humains, comprennent: rapidité dans la prolifération cellulaire et la réparation tissulaire ; une matrice en 3D pleinement fonctionnel ; parfaites hélices alpha de collagène ; liaisons cellulaires élevées.

Le RhCollagène a une fonction biologique supérieure par rapport au collagène dérivé de tissu animal ou humain et a un certain nombre de caractéristiques physiques utiles, y compris la stabilité thermique ou la résistance à la décomposition à haute température, et une triple hélice primitive, selon des publications. La structure en triple hélice du collagène de type I est formée lorsque deux chaînes protéiques α 1 et une chaîne protéique α 2 s'enroulent ensemble selon un axe commun. Le collagène de type II est formé de trois chaines α 1 (II). Dans la formation de rhCollagène, cette structure est réalisée sans modifications qui peuvent conduire à des défauts dans la structure de la triple hélice, conduisant ainsi à une triple hélice immaculée identique à la forme trouvée dans la nature. Une triple hélice immaculée permet une liaison supérieure, ce qui accélère la prolifération des cellules humaines primaires, fibroblastes et tissu fibro-cartilage.

Dans tous les types de cellules testés, la prolifération cellulaire était significativement meilleure dans les échafaudage faits de rhCollagène que dans les échafaudages disponibles dans le commerce faits de collagène bovin. La prolifération cellulaire accélérée obtenue avec le rhCollagen entraîne une cicatrisation plus rapide, et une régénération tissulaire de meilleure qualité.

Le rhCollagène pur n'induit pas de réponse immunogène, alors que les impuretés provenant de la source de collagène dérivé de tissu peuvent conduire au rejet du système immunitaire. Des études in vitro réalisées dans le cadre d'une collaboration académique ont démontré que le rhCollagène incubé avec les macrophages THP1 activés produit des taux significativement plus bas de cytokines inflammatoires par rapport au collagène bovin. Cela démontre que le collagène dérivé de l'animal peut provoquer une réaction du corps étranger non observée avec rhCollagen, ce qui retarde la cicatrisation et augmente les cicatrices. En outre, il n'y a pas d'effets secondaires potentiels dans la croissance des tissus, car il n'y a pas de résidus de croissance. Les facteurs proviennent des tissus extraits. En outre, avec le collagène dérivé de tissu, il est possible que l'animal ou l'humain à partir duquel le collagène a été produit ait été infecté par un virus, un prion ou un autre agent pathogène. Avec le rhCollagen, il n'y a aucun risque de transmission de maladies et de pathogènes.

Le RHCollagen est synthétisé par plusieurs gènes humains dans des plants de tabac produisant des molécules pures qui sont répétables et identiques au collagène humain de type I ou II ; il est plus homogène que le collagène dérivé de sources tissulaires animales ou humaines. Le haut niveau d'homogénéité du rhCollagen permet la formulation de concentrations extrêmement élevées de collagène soluble de type I ou II en triple hélice, jusqu'à 150-200 mg / ml, ce qui est au moins 10 à 100 fois plus élevé que la concentration obtenue avec du collagène dérivé de tissu. La forte concentration de collagène monomère homogène est particulièrement importante lorsque des fibres de collagène solides sont nécessaires pour les échafaudages 3D. L'homogénéité du rhCollagen permet d'élaborer des produits cohérents et reproductibles avec un taux de dégradation contrôlé qui peut être optimisé pour l'indication ciblée

Par rapport au collagène dérivé de tissu, les membranes de rhCollagène ont montré une meilleure stabilité thermique, une meilleure résistance à la traction due à l'alignement des fibres de collagène, et des niveaux plus élevés de transparence.

Le Rh Collagène est autorisé pour son utilisation humaine par l'EMA (Europe) et la FDA (USA).

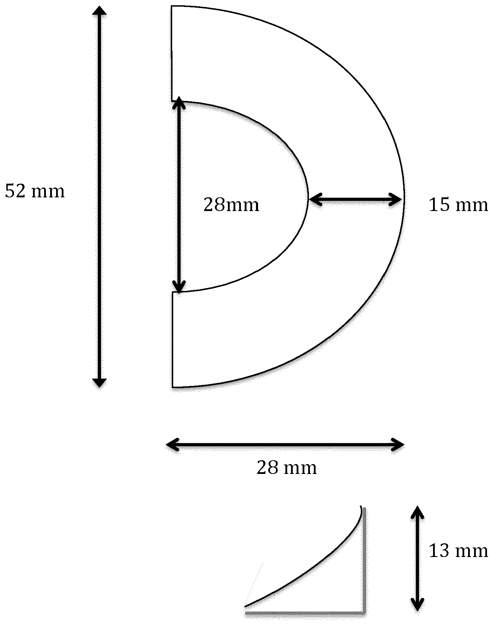

Selon un mode de réalisation, le scaffold méniscal est arqué : l'implant méniscal a une seule forme aussi bien médial que latéral, en forme de C ou croissant, dont la section est en forme de coin; et dont l'élasticité permet de le modifier pour s'adapter à la forme du ménisque traité médial ou latéral. Il est concave à sa partie supérieure et plane dans sa partie inférieure. La figure 1 note les dimensions antéro-postérieure, et la figure 2 celles médio-latéral et l'épaisseur. Il est plus épais et plus large que les implants de l'art antérieur. Sa section est en forme de coin et sa surface est plane coté tibial et incurvée coté fémoral. L'implant méniscal possède des systèmes de sutures incorporés, permettant une suture plus rapide sur le mur méniscal ou la capsule périphérique.

Dans un mode de réalisation, l'invention est un implant scaffold méniscal composite, indiqué et utilisé dans l'arthrose terminale du genou grade 3 ou 4 avec perte d'au moins 50% du ménisque, et selon critères d'inclusion (âge, poids, performance physique), pour permettre la régénération de cartilage combinée à la thérapie cellulaire ; et constitué d'uns structure mécanique en polyuréthane de haut poids moléculaire et autres polymères PCA et PLGA, en mousse et poreux ; et d'une structure biologique stimulant la colonisation cellulaire et de tissus fibrocartilage du scaffold, comportant des couches de collagènes, Rhcollagènes, collagène recombinant humain, non animal, tiré de la plante de tabac.

L'un des modes de réalisation de l'invention concerne des implants méniscaux biocompatibles fabriqués à partir des mousses poreuses de polyuréthane, de polymères et de collagène recombinant de plante du présent procédé; ils se dégradent après l'implantation et les produits de dégradation sont biocompatibles. Des biopsies à 12 mois ont été réalisées, montrant un tissu en cours de maturation avec une différenciation fibrochondrocytaire et des faisceaux de collagène organisés. Des études cliniques ont été publiées, montrant l'incorporation de l'implant méniscal polyuréthane Actifit (Ortek) à l'IRM et contrôle arthroscopique second look, et les bons résultats cliniques (Verdonk, Assor). Mais des dégradations partielles et fragments de polyuréthanes ; et une destruction résorption spontanées du scaffold collagène bovin Menaflex, ont été relevés ainsi qu'une insuffisance de colonisations cellulaires, soulignant le besoin d'un implant scaffold méniscal adapté au milieu articulaire du genou.

Dans un mode de réalisation, l'implant méniscal composite mécanique et biologique est fabriqué par une imprimante 3D.

**Propriétés mécaniques.** La résistance à la pression est un paramètre important du scaffold méniscal : elle détermine la capacité de l'implant à résister à la compression articulaire, et de garder les pores (porosité de l'implant, lieu de la colonisation cellulaire) ouvert, pour permettre la croissance du tissu fibro-cartilage et sa maturation. Le stress dans une articulation normale est de 2-3 Mpa ; avec un pic de stress de 5-6 Mpa après méniscectomie. Sous une contrainte de 2 Mpa, les implant actuels Actifit et Menaflex sont écrasés, détruits, empêchant toute colonisation cellulaire par le collapsus des pores, et se résorbent. Ces implants nécessitent des suites post-opératoires difficiles sur 2 à 3 mois, avec réduction importante de la flexion et de l'appui, entraînant le plus souvent une raideur avec adhérences articulaires douloureuses préjudiciables à la fonction à long terme de l'articulation. Il est intéressant que ce scaffold ait une plus grande résistance pour faciliter la mobilité et la marche post-opératoire précoce, et la bonne fonction articulaire du genou. Un module de compression de plus de 450 Kpa , et une porosité suffisante sont nécessaires pour favoriser la croissance tissulaire. Le module de compression est lié à la densité et à la porosité de l'implant méniscal, ainsi qu'à la densité de sa périphérie pour résister aux forces de traction

Dans un autre mode de réalisation, le polyuréthane, ses polymères associés, et son composite associé de collagène recombinant dans la présente invention, a un poids moléculaire moyen d'environ 240 kg / mole ou plus, avec un module de compression compris de préférence entre proche de 1 à 2 MPa (1000 à 2000 kPa) , sachant qu'en milieu liquide à 37° de température, le module de compression baisse de 43%.

II a une résistance à la déchirure d'environ 20-25 N / mm ou plus; il a une flexibilité (déformation à la rupture) d'environ 400 à 500% ; et une densité d'environ 0,30 / cm3 ou plus.

Pourcentage de la porosité : 77 à 84% ; permettant une bonne croissance tissulaire ; avec une taille des pores de 400 à 800 nm.. Pour favoriser la croissance du tissu, les échafaudages poreux ont une structure poreuse interconnectée qui peut être créée par lixiviation particulaire. Le diamètre de l'interconnexion entre les pores est préférable de plus de 30 µm.

Ces propriétés avantageuses sont en partie dues au poids moléculaire élevé des polymères dans la mousse et du collagène recombinant ; et en partie à cause de l'interconnectivité des polymères dans la mousse.

Le polyuréthane contient deux types de liaisons qui sont sensibles à l'hydrolyse: les liaisons ester et les liaisons uréthane. Les liaisons uréthane sont plus résistantes au clivage hydrolytique. On s'attend donc à ce que le clivage hydrolytique des liaisons ester de poly (ε-caprolactone) se produise d'abord in vivo.

Étant donné que la pénétration de tissu dans l'implant devrait être complète après 3-4 mois, il est souhaitable que l'implant conserve ses propriétés mécaniques pendant au moins 6-9 mois. Après l'implantation, le poids moléculaire du polyuréthane diminue à la suite de l'hydrolyse du polymère dans le corps. Il est préféré pour la fonctionnalité des implants composite en polyuréthane-polymères et en Collagène de plante (Collplant) de la présente invention que l'implant scaffold poreux conserve ses propriétés mécaniques pendant au moins six mois. L'adjonction par cross linking et lyophilisation du Collagène permet de conserver les propriétés mécaniques plus de 6 mois et de faciliter la colonisation rapide de tissus cartilagineux dans les pores de l'implant.

Parmi les propriétés mécaniques décrites ci-dessus, la dépendance à la force de déchirement sur le poids moléculaire est la plus critique. Au-dessus d'un poids moléculaire de 100 kg / mol, la résistance au déchirement avec le composite collagène est de 20-25 N / mm. Par conséquent, après 3 mois d'implantation, le polymèrecollagène du scaffold poreux doit avoir un poids moléculaire supérieur à 100 kg / mol. La dégradation in vivo est dominée par l'hydrolyse et, par conséquent, comparable à la dégradation in vitro à 37°C. Quatre mois de dégradation du polymère entraînent une diminution du poids moléculaire d'environ 7 kg / mol. Par conséquent, le scaffold poreux composite doit avoir un poids moléculaire moyen supérieur à environ 240 kg/mole pour que l'implant conserve les caractéristiques physiques souhaitées après avoir été implanté pendant trois mois. Le terme biocompatible signifie que l'implant de la présente invention ainsi que les débris d'usure et les matériaux générés pendant la dégradation in vivo ne provoquent pas une réponse immunitaire substantielle, une sensibilité, une irritation, une cytotoxicité ou une génotoxicité.

### Fabrication-Constitution

Selon un mode de réalisation, la structure mécanique de polyuréthane combinée avec PCA polycarbonate et PLGA polyglicolactide, est fabriquée par un procédé sans trace de catalyseur comprenant :
(1) La réaction du 1,4-butanediol, avec un composé oxygéné l'ε-caprolactone de poids moléculaire d'environ 1700 g/mole pour former un macrodiol par polymérisation par ouverture de cycle, poids moléculaire compris entre 1500 et 3000 g / mol ;
(2) Traiter le macrodiol avec un diisocyanate, - formule OCN-R-OCN, où R est un alkylène en C3 à C6, aliphatiques et cycloaliphatiques - pour obtenir un macrodiisocyanate ;
(3) Faire réagir le macrodiisocyanate avec un allongeur de chaîne diol, le 1,4-butanediol, dans un rapport molaire de préférence 1,00: 1,01 à 1,00: 1,03.
(4) Faire réagir Le PLGA (50-50%) ;
(5) et le PCA uréthane, selon procédé de polymérisation en plusieurs étapes, à bonne biocompatibilité et propriétés mécanique, est synthétisé par polymérisation en utilisant les poly (1,6-hexanediol) carbonates diols (PCDL) segment, 4,4'-méthylènebis (cyclohexylisocyanate) (H ₁₂MDI), le 1,6-hexaméthylène diisocyanate (HDI) et le 1,4-butanediol (BDO) en tant que segment dur avec le dilaurate de dibutylétain en tant que catalyseur, et que l'homme de l'art comprend les processus.
(6) Les polymères sont éventuellement renforcés en périphérie par des fibres de type polyuréthane urée ou autres fibres utilisées en application médicale.
(7) Un hydrogel physique supramoléculaire peut être associé pour augmenter les propriété mécanique et d'absorption des pressions axiales.
(8) Inclure par procédé chimique chimique cross-linking ou fusion, ou en impression 3D, le collagène recombinant humain de plante
(9) Dans un autre mode de réalisation, le procédé de préparation de la porosité du scaffod du polyurethane, du PCA et du PLGA ou mousse poreuse, comprenant les étapes consistant à: (1) Préparer une solution de polyuréthane-polymères, tel que préparé selon le procédé dans un solvant approprié où ils sont solubles, de préférence DMSO, diméthylacétamide, ou autres solvants : DMF, le NMP, le crésol, le 1,4-dioxane, le chloroforme, à une température de fusion ; (2) combiner la solution avec un non-solvant, de préférence de l'eau, pour obtenir une solution, de préférence la quantité de non-solvant ajoutée à la solution est comprise de 5% à 10%; (3) ajouter un matériau porogène non soluble dans le solvant, un sel ou NaCl, ou un sucre de préférence (glucose, saccharose), pour obtenir un mélange visqueux ; (4) verser le mélange visqueux dans un moule et / ou le refroidir, dans n'importe quel ordre pour obtenir un matériau moulé; et (5) laver le matériau moulé avec un non-solvant dans lequel le polyuréthane, les polymères et le collagène recombinant, lié par cross linking et lyophilisation, constituant le scaffold est insoluble, mais dans lequel le matériau porogène peut être dissous pour obtenir une mousse matière poreuse ; le matériau lavé est traité thermiquement ; le niveau de température et sa durée influent sur les propriétés du scaffold. Un procédé préféré pour préparer des échafaudages poreux de la présente invention comprend le procédé tel que décrit dans la demande de brevet US publiée US 2007/0015894.

La mousse biocompatible de la présente invention fournit un échafaudage pour la formation de cartilage dans le remplacement du ménisque détruit par l'arthrose du genou, ou réséqué, et aide à la régénération de cartilage de l'arthrose. Dans les modes de réalisation décrits du composite de polymère polyuréthane et collagène recombinant, des modes de réalisations adaptés et améliorés apparaîtront à l'homme de l'art. Il apparaîtra à l'homme de l'art que de nombreuses modifications, à la fois sur les matériaux et sur les méthodes, peuvent être pratiquées sans sortir du cadre de l'invention.

L'utilisation d'une diamine comme allongement de chaîne peut conduire à des polyuréthanes urées ayant de meilleures propriétés mécaniques, par rapport aux polyuréthanes basés sur un allongeur de chaîne diol. Mais avec le procédé de l'invention, les polyuréthanes peuvent être synthétisés avec d'excellentes propriétés mécaniques.. L'utilisation d'un diol comme allongeur de chaîne à la place d'une diamine présente l'avantage que le polyuréthane produit est plus facile à mettre en oeuvre.

L'avantage des implants poreux est que la croissance du tissu est possible dans les pores. Pour favoriser la croissance du tissu, les échafaudages poreux ont de préférence une structure poreuse interconnectée qui peut être créée par lixiviation particulaire.

Le diamètre de l'interconnexion entre les pores est préférable de plus de 30 µm. En général, les mousses à utiliser en tant qu'échafaudages poreux dans des implants corporels peuvent être fabriquées de diverses manières connues dans la technique, telles que la lyophilisation / la lixiviation particulaire, décrite plus haut.

Le renforcement par cross linking et lyophilisation ou impression 3D, par des collagènes humains recombinant de plante peut créer un échafaudage poreux encore plus fort. Dans le procédé de préparation d'un échafaudage poreux tel que décrit ici, des fibres de RHcollagène recombinant de plantes, peuvent être ajoutées au mélange homogène préparé à l'étape 2) des procédés de l'invention, par cross-linking ou impression 3D.

Les fibres de RHcollagène recombinant de plante peuvent être orientées de manière à simuler la structure fibreuse native du collagène.

Dans un autre mode de réalisation, de telles fibres de collagène sont incorporées dans l'échafaudage poreux tout en s'étendant à partir de l'échafaudage poreux.

D'autres fibres appropriées peuvent être utilisées : les urées de polyuréthanne ou de polyuréthanne préparées selon les procédés décrits ici ou tout autre polyuréthane, polyuréthane-urée, ou fibre utile dans des applications médicales.

Des structures poreuses avec des porosités supérieures ayant une porosité de 70 ou 80% pourraient être obtenus. Le refroidissement à une température d'environ 20 ou -18 ° C donne de bons résultats.

Le solvant ou le mélange de solvants et le matériau formant des pores doivent être lavés à une température inférieure à la température de fusion du diluant de polymère. Des agents de lavage appropriés pour des solvants tels que le DMSO, le NMP, le DMF et le dioxane mélangés avec de l'eau non-solvant, de l'éthanol ou de l'eau et de l'éthanol. Lorsque l'on utilise des non-solvants polaires tels que l'éther diéthylique, l'hexane, l'éthanol est un agent de lavage approprié. L'eau peut toujours être un bon agent de lavage, mais doit être mélangée avec une certaine quantité d'éthanol pour assurer le mélange du non-solvant dans les agents de lavage. Lorsque des solvants tels que le chloroforme sont utilisés et par exemple l'éthanol, l'hexane ou le pentane sont utilisés comme non-solvants, et un agent de lavage approprié est l'éthanol. Dans la seconde étape, le matériau porogène est éliminé par lavage. Il est préférable que l'agent porogène soit soluble dans l'agent de lavage mais que le polymère ne se dissolve pas dans l'agent de lavage (non-solvant pour le polymère). Un agent de lavage approprié pour éliminer par exemple le saccharose ou le NaCl, le saccharose ou le glucose est l'eau. Le mélange de solvants et le mélange formant des pores peuvent également être lavés immédiatement lorsqu'ils sont tous deux solubles dans l'agent de lavage. Le procédé de fabrication d'échafaudages poreux est particulièrement approprié pour préparer des échafaudages poreux des polyuréthanes (fabriquées selon le procédé).

L'échafaudage poreux lui-même peut incorporer des facteurs de croissance ou d'autres agents qui amélioreront la formation du cartilage.

**Le composite Polyuréthane-Polymères-Rh Collagène.** L'implant méniscal est construit avec le polyuréthane, avec deux autres polymères PCA et PLGA, et le Rh Collagène, par différentes méthodes: par trempage simple avec manipulations chimiques, cross linking et lyophilisation, technique préférée, pour faire inclure (accrocher) le collagène dans les pores du polyurethane-polymères de l'implant méniscal; ou par fusion par température et compression, ou impression 3D.

Une imprimante 3D permet la fabrication de l'implant méniscal selon la forme décrite ci-après, avec un seul élément pour l'interne et l'externe.

Cet implant composite est supérieur à l'implant polyuréthane-polymères isolé : il possède une résistance plus élevée à la traction et à la pression ; une plus haute résistance à la déchirure et à la traction des points de suture ; une plus grande souplesse.

Surtout, l'implant méniscal composite polyuréthane-Rh Collagène garde son poids moléculaire plus de trois mois ; se colonise plus rapidement par du tissu cartilage et protéoglycans grâce à la présence et la structure homogène du collagène, et permet un bon résultat clinique à long terme et plus régulier, avec une restauration du ménisque et du cartilage lorsque l'implant méniscal est utilisé dans l'arthrose du genou évoluée grade 3 ou 4.

Sur la base de ces résultats cliniques à long terme du polyuréthane, et des tests du collagène issu de plantes, l'implant composite de ménisque d'échafaudage scaffold étudié constitue une option de traitement sûre et viable pour les déchirures irréparables et le remplacement du ménisque, et du traitement de l'arthrose grave du genou. De plus, le matériau composite avec du collagène a une plus grande tendance à résister à l'infection avec l'apport rapide de tissus et de cellules, que le polyuréthane seul.

Les dessins annexés illustrent l'invention : représentent en coupe, le dispositif de l'invention et ses cotes.

Dans un autre mode de réalisation, des fils de suture sont prépositionnés et inclus chaque cm pour faciliter la pose

### Les Tests mécaniques et de biocompatibilité

1. Détermination du poids moléculaire du polyuréthane et des polymères. Le poids moléculaire des polymères est déterminé en utilisant une Chromatographie par perméation de gel (GPC) (Shimadzu T030845) avec des étalons de polystyrène et en utilisant du LiBr 0,01 M dans du DMF
2. Résistance à la déchirure et flexibilité de la mousse. Des tests sont effectués sur des échantillons d'une épaisseur d'environ 10 mm. Des suture 2-0 ont été positionnées à 3-5 mm du bord de l'échantillon, placés dans les pinces du testeur de traction (Instron 3342). La vitesse de la tête transversale était de 10 mm / min. La résistance au déchirement est calculée comme suit: la force maximale (N) est divisée par l'épaisseur de l'échantillon d'essai. La résistance à la déchirure est d'au moins 3,0 N / mm, de préférence de 20 à 25 N / mm.
3. La flexibilité a été calculée comme suit: le déplacement à la rupture divisé par la distance de la suture au bord du matériau de l'implant.
4. Densité de la mousse : Sur des échantillons d'une épaisseur d'environ 8-10 mm, les dimensions et le poids de l'échantillon ont été déterminées et la densité (g / cm3) a été calculée à partir de la masse (g) et du volume (cm3) du matériau.
5. Dégradation du scaffold poreux. Il est prévu que la dégradation in vivo se produise sur une période moyenne de 4 ans. Selon les tests de l'art passé et en particulier les tests du brevet du polyuréthane Orteq, les changements de poids moléculaire pendant la dégradation in vitro à 37 ° C dans un tampon phosphate ont montré, après 1,5 ans, que le poids moléculaire a diminué jusqu'à 50% de son poids moléculaire d'origine.
6. Cytotoxicité de l'implant. Elle a été démontré non toxique dans la littérature et en particulier les tests du brevet Orteq. Un segment d'un implant polymère a été extrait et l'extrait a été mis en contact avec des cellules. La lyse des cellules (mort cellulaire), l'inhibition de la croissance cellulaire et d'autres effets sur les cellules causés par l'extrait ont été déterminés. L'implant est passé et il n'y avait aucune évidence de lyse cellulaire. Dans une autre étude, le PCL e le PLGA ont montré la faiblesse de la réponse inflammatoire, surtout pour le PCL (22)
7. De même, absence de réaction allergique ou d'irritation après injection du produit par voie intra dermique du lapin ; ou de toxicité systémique après injections intra-péritonéale sur des souris.
8. Génotoxicité sur implant: mutation inverse bactérienne. Le test a été réalisé pour évaluer le potentiel mutagène de l'implant poluréthane brevet Orteq. Les bactéries ont été exposées aux extraits d'implants ; la mutation a été déterminée après incubation. L'implant a passé, aucun effet toxique n'a été observé.
9. Génotoxicité sur implant: test d'aberration chromosomique dans des cellules de mammifères in vitro. Le test a été réalisé pour évaluer les propriétés clastogènes potentielles sur les chromosomes des lymphocytes humains. Des cultures de lymphocytes humains ont été exposées à l'implant extrait dans du NaCl à 0,9%. Une étude préliminaire a été réalisée sans le système d'activation métabolique afin de déterminer la toxicité possible de cinq concentrations de l'extrait. La concentration non toxique la plus élevée (40 µL d'extrait / mL de milieu de culture) a été testée. Après la période de contact, les cultures ont été traitées afin d'effectuer la préparation des chromosomes. La détection des aberrations a été réalisée en observant les chromosomes. L'implant est passé, aucun effet n'a été observé.
10. Génotoxicité sur implant: micronoyau de moelle osseuse de souris. Le test a été réalisé pour évaluer la puissance mutagène après des injections intra-péritonéales chez des souris des extraits d'implants. Le test et les groupes témoins négatifs ont reçu une injection intra-péritonéale pendant deux jours, tandis que les souris témoins positives ont reçu une seule injection intra-péritonéale de cyclophosphamide le deuxième jour. Des souris ont été observées immédiatement après l'injection pour l'état de santé général et pour tout effet indésirable. Au jour 3, toutes les souris ont été pesées et terminées. Les fémurs ont été excisés, la moelle osseuse a été extraite et des préparations de frottis en double ont été réalisées sur chacune d'elles. Des cellules de mammifères ont été exposées à l'implant extrait dans du NaCl à 0,9% et dans de l'éthanol à 96%. La mutation a été déterminée après incubation. L'implant a passé, il n'y avait pas d'effets mutagènes / toxiques observés.
11. Etude de toxicité subchronique combinée et étude de tolérance locale sur un matériau d'implant et un implant accéléré (segments de polyuréthanne). Des produits de dégradation d'implant accélérés ont été fabriqués comme suit. Le matériau d'implant en poudre a été soumis à du HCl 9M pendant 3 jours. Le matériau restant (les segments durs) a été isolé à travers plusieurs étapes de lavage, centrifugé et séché. Une purification supplémentaire a été effectuée par lavage avec de l'eau apyrogène et finalement lavage avec de l'éthanol à 96% (qualité pharmaceutique). Après séchage dans un four à vide, les segments durs ont été pulvérisés avec un moteur et un pilon. L'analyse Malditov- et 1H-RMN a montré que la dégradation du segment mou était efficace et que les segments durs étaient principalement restés. L'analyse MEB était trop grande et non représentative de la taille réelle des segments durs (les petites particules regroupées à la suite du processus de lavage et de séchage). Par conséquent, une procédure de sonication a été réalisée dans une solution saline à 0,9%, ce qui a donné une dispersion laiteuse dans laquelle, au repos, aucun sédiment n'a été observé. L'analyse SEM a révélé que 98% des particules dans la dispersion laiteuse étaient représentatives de la taille des segments durs qui seraient attendus après dégradation in vivo des segments mous (70 à 130 nm). La dispersion laiteuse (0,4 ml) a été injectée dans l'espace sous-cutané dorsal des rats et le site a été marqué par tatouage à l'encre pour identifier le site d'injection à la nécropsie. De plus, des disques de matériel d'implant pesant 90 ± 2 mg d'une épaisseur de 2,5 ± 1,1 mm ont été stérilisés et implantés dans un côté du dos de 10 rats mâles et de 10 rats femelles (de l'autre côté du dos 2 mL de 0,9 % NaCl a été injecté en tant que contrôle). Un groupe témoin a reçu un disque de polyéthylène haute densité. Les rats ont été observés immédiatement après l'implantation et tous les jours après pour détecter la mortalité ou la morbidité et tout signes cliniques normaux. Le poids corporel et la prise alimentaire ont été enregistrés chaque semaine. A la fin de l'intervalle d'implantation (13 semaines), des échantillons de sang ont été prélevés pour l'hématologie et la chimie clinique et les rats ont été soumis à une nécropsie sous-acroscopique et à un examen microscopique d'organes sélectionnés et de sites implantés. Aucune mortalité ou signe clinique pouvant être lié à un effet toxique des implants n'a été observé. Le matériau d'implant dégradé (segments durs) a été absorbé par les macrophages.
12. Etude combinée de toxicité chronique et de tolérance locale sur un matériau d'implant et un implant accéléré (segments de polyuréthane), 26 semaines. Un groupe de rats a été implanté avec l'implant de la présente invention. Un groupe a été injecté avec l'implant dégradé accéléré (agglomérats de segments de polyuréthane de tailles 70-130 nm) comme décrit ci-dessus. Un groupe témoin de 10 rats mâles et 10 rats femelles a reçu un disque de polyéthylène haute densité. Les rats ont été observés immédiatement après l'implantation, puis quotidiennement pour détecter la mortalité ou la morbidité et tout signe clinique anormal. Le poids corporel et l'apport alimentaire ont été enregistrés une fois par semaine. À la fin de l'intervalle d'implantation (26 semaines), des échantillons de sang ont été prélevés pour l'hématologie et la chimie clinique et les rats ont été soumis à une nécropsie macroscopique et à un examen microscopique des organes sélectionnés et des sites implantés. L'implant a passé, il n'y avait aucun signe clinique d'effet toxique. Le matériel d'implant dégradé (segments durs) a subi une phagocytose par les macrophages.
13. Analyse des débris d'usure. Le stress que subit le genou est très élevé et des particules de l'implant peuvent se détacher de l'implant. Un test de débris d'usure pour les implants polymère polyuréthane a été effectué dans le modèle de genou de lapin pour montrer la sécurité des débris de particules. Ce test a été réalisé pour évaluer la tolérance locale des débris d'usure résultant de l'implantation, quatre semaines après une injection intra-articulaire dans le genou de lapin. Quatre semaines après l'injection, chaque genou a été disséqué, ouvert et examiné et un examen macroscopique de chaque compartiment du genou a été réalisé. Il n'y avait pas de signes de douleur ou de gonflement et il n'y avait pas d'accumulation de liquide synovial.
14. Implantation chez les moutons ou chèvres : les tests sur animaux vivants vont être réalisés pour évaluer la présente invention de scaffold composite polyuréthane-polymère et collage recombinant de plante. Selon la procédure suivante, dont les résultats seront publiés.
   La perte du capital méniscal, après méniscectomie pour lésions graves, ou du fait du processus arthrosique avec perte de grade 3 ou 4 du cartilage fémoro-tibial (médial et/ou latéral), est responsable de l'aggravation de l'arthrose du genou, (23) :Scheller et al., Arthroscopy 17: 946-52 (2001). Les implants poreux de la présente invention vont être étudiés pour évaluer leurs performances à long terme après implantation dans un modèle de méniscectomie ovine partielle sur la chèvre. Dix chèvres matures sont soumis à une méniscectomie totale unilatérale. Chez 5 animaux, la méniscectomie totale a été remplacée par un échafaudage. Le résultat principal mesuré était le classement histologique des lésions cartilagineuses sur le plateau tibial et le condyle fémoral. Résultats secondaires: (i) apparence générale du genou, (ii) coefficient de frottement de l'échafaudage en fonction du temps, (iii) évidence de pénétration tissulaire dans l'échafaudage, et (iv) caractéristiques de transfert de charge en fonction du temps. Trois mois après l'implantation, 5 genoux implantés sur un échafaud et 5 genoux partiellement méniscectomisés sont analysés. Trois mois plus tard, la colonisation cellulaire de l'échafaudage sera analysée. Les dommages du cartilage sont analysés ; pour démontrer que la protection du cartilage est assurée par l'échafaudage par rapport à la méniscectomie partielle sans implantation d'échafaudage.
15. Un projet de demande d'essai clinique chez l'homme sera déposé aux autorités sanitaires.

## Revendications

1. Echafaudage composite pour implant méniscal comportant une structure polymérique poreuse et un collagène associé à la structure polymérique poreuse, **caractérisé en ce que** :
- la structure polymérique poreuse comporte un polyuréthane (PU), un polycarbonate-uréthane (PCA) et un poly(acide lactique-co-glycolique) (PLGA) ;
- le collagène est un collagène humain recombinant de type I ou II produit à partir d'une plante, notamment de la plante de tabac.

2. Echafaudage composite selon la revendication 1, **caractérisé en ce qu'il** est biocompatible et biodégradable.

3. Echafaudage composite selon la revendication 1 ou 2, **caractérisé en ce qu'il** est colonisable par du fibrocartilage avec lequel il est en contact.

4. Echafaudage composite selon l'une des revendications 1 à 3, **caractérisé en ce que** le polymère poly(acide lactique-co-glycolique) (PLGA) a un rapport entre acide lactique et acide glycolique de 50/50.

5. Echafaudage composite selon l'une des revendications 1 à 4, **caractérisé en ce que** le polyuréthane (PU) est le produit d'un procédé, sans trace de catalyseur, comprenant :
- la réaction du 1,4-butanediol avec de l'ε-caprolactone de poids moléculaire d'environ 1700 g/mol pour former un macrodiol ayant un poids moléculaire compris entre 1500 et 3000 g/mol ;
- la réaction du macrodiol obtenu ci-dessus avec un diisocyanate pour obtenir un macrodiisocyanate ;
- la réaction du macrodiisocyanate obtenu ci-dessus avec un allongeur de chaîne, lequel allongeur de chaîne est du 1,4-butanediol.

6. Echafaudage composite selon l'une des revendications 1 à 5, **caractérisé en ce qu'**il possède une porosité suffisante favorisant la colonisation de l'échafaudage composite par le fibrocartilage avec lequel il est en contact et la croissance dudit fibrocartilage.

7. Echafaudage composite selon l'une des revendications 1 à 6, **caractérisé en ce qu'**il est réalisé sous une forme unique imitant la forme arquée du ménisque, latéral ou médial, du genou.

8. Echafaudage composite selon l'une des revendications 1 à 7, pour une utilisation comme implant dans des applications de remplacement de ménisque du genou, et/ou de traitement de l'arthrose du genou, notamment de l'arthrose grade 3 et 4 du genou.

9. Implant de remplacement de ménisque du genou, et/ou de traitement de l'arthrose du genou, notamment de l'arthrose grade 3 et 4 du genou, **caractérisé en ce qu'**il comprend un échafaudage composite selon l'une des revendications 1 à 7.

10. Implant selon la revendication 9, **caractérisé en ce qu'**il est réalisé sous une forme en C unique utilisable aussi bien pour le ménisque latéral du genou que pour le ménisque médial du genou.

11. Procédé de fabrication d'un échafaudage composite selon l'une des revendications 1 à 7, comprenant au moins les étapes consistant à :
- fournir une structure polymérique poreuse telle que définie à l'une des revendications 1 à 7 ;
- appliquer du collagène à la structure polymérique poreuse par une technique choisie parmi les techniques de trempage, de cross-linking et lyophilisation ou d'impression en trois dimension (3D) pour obtenir l'échafaudage composite, le collagène étant un collagène humain recombinant de type I ou II produit à partir d'une plante, notamment de la plante de tabac.

12. Procédé de fabrication selon la revendication 11, dans lequel le collagène est appliqué à la structure polymérique poreuse par cross-linking et lyophilisation.

13. Procédé de fabrication selon la revendication 11, dans lequel le collagène est appliqué à la structure polymérique poreuse par impression en trois dimensions (3D).

## Patentansprüche

1. Kompositgerüst für Meniskusimplantat, das einen porösen Polymeraufbau sowie ein Kollagen aufweist, das dem porösen Polymeraufbau zugehörig ist, **dadurch gekennzeichnet, dass:**
- der poröse Polymeraufbau ein Polyurethan (PU), ein Polycarbonaturethan (PCA) und ein Polylactid-co-Glycolid (PLGA) aufweist;
- das Kollagen ein rekombinantes menschliches Kollagen Typ I oder II ist, das aus einer Pflanze, insbesondere der Tabakpflanze, hergestellt ist.

2. Kompositgerüst nach Anspruch 1, **dadurch gekennzeichnet, dass** es biokompatibel und biologisch abbaubar ist.

3. Kompositgerüst nach Anspruch 1 oder 2, **dadurch gekennzeichnet, dass** es mit dem Faserknorpel, den es berührt, besiedelbar ist.

4. Kompositgerüst nach einem der Ansprüche 1 bis 3, **dadurch gekennzeichnet, dass** das Polylactid-co-Glycolid-Polymer (PLGA-Polymer) ein Verhältnis von Milchsäure zu Glycolsäure von 50/50 hat.

5. Kompositgerüst nach einem der Ansprüche 1 bis 4, **dadurch gekennzeichnet, dass** das Polyurethan (PU) das Produkt eines Verfahrens ohne Spur eines Katalysators ist, umfassend:
- die Reaktion von 1,4-Butandiol mit ε-Caprolacton mit einer Molekülmasse von ungefähr 1700 g/mol zur Bildung eines Makrodiols mit einer Molekülmasse zwischen 1500 und 3000 g/mol;
- die Reaktion des zuvor erhaltenen Makrodiols mit einem Diisocyanat zum Erhalten eines Makrodiisocyanats;
- die Reaktion des zuvor erhaltenen Makrodiisocyanats mit einer Kettenlänge, wobei die Kettenlänge die von 1,4-Butandiol ist.

6. Kompositgerüst nach einem der Ansprüche 1 bis 5, **dadurch gekennzeichnet, dass** es eine Porosität besitzt, die ausreichend hoch ist, dass sie die Besiedlung des Kompositgerüsts mit dem Faserknorpel, den es berührt, und das Wachstum des Faserknorpels begünstigt.

7. Kompositgerüst nach einem der Ansprüche 1 bis 6, **dadurch gekennzeichnet, dass** es in einer einzigartigen Form ausgeführt ist, die die gebogene Form des Außenoder Innenmeniskus des Knies nachahmt.

8. Kompositgerüst nach einem der Ansprüche 1 bis 7, für eine Verwendung als Implantat in Anwendungen zum Ersatz des Meniskus im Knie und/oder zur Behandlung der Kniearthrose, insbesondere bei Kniearthrose Grad 3 und 4.

9. Implantat zum Ersatz des Meniskus im Knie und/oder zur Behandlung der Kniearthrose, insbesondere bei Kniearthrose Grad 3 und 4, **dadurch gekennzeichnet, dass** es ein Kompositgerüst nach einem der Ansprüche 1 bis 7 umfasst.

10. Implantat nach Anspruch 9, **dadurch gekennzeichnet, dass** es in einer einzigartigen C-Form ausgeführt ist, die sowohl für den Außenmeniskus des Knies als auch für den Innenmeniskus des Knies einsetzbar ist.

11. Verfahren zur Herstellung eines Kompositgerüsts nach einem der Ansprüche 1 bis 7, das mindestens folgende Schritte umfasst:
- Bereitstellen eines porösen Polymeraufbaus nach einem der Ansprüche 1 bis 7;
- Aufbringen von Kollagen auf den porösen Polymeraufbau mit einem Verfahren, das ausgewählt ist aus Verfahren zum Eintauchen, Vernetzen und Gefriertrocknen oder 3D-Druck (3D) zum Erhalten des Kompositgerüsts, wobei das Kollagen ein rekombinantes menschliches Kollagen Typ I oder II ist, das aus einer Pflanze, insbesondere der Tabakpflanze, hergestellt ist.

12. Herstellungsverfahren nach Anspruch 11, wobei das Kollagen mittels Vernetzen und Gefriertrocknen auf den porösen Polymeraufbau aufgebracht wird.

13. Herstellungsverfahren nach Anspruch 11, wobei das Kollagen mittels 3D-Druck (3D) auf den porösen Polymeraufbau aufgebracht wird.

## Claims

1. Composite scaffold for a meniscal implant, comprising a porous polymeric structure and a collagen combined with the porous polymeric structure, **characterized in that:**
- the porous polymeric structure comprises a polyurethane (PU), a polycarbonate-urethane (PCA) and a poly(lactic-co-glycolic acid) (PLGA);
- the collagen is a recombinant human collagen type I or II produced from a plant, in particular from the tobacco plant.

2. Composite scaffold according to Claim 1, **characterized in that** it is biocompatible and biodegradable.

3. Composite scaffold according to Claim 1 or 2, **characterized in that** it can be colonized by fibrocartilage with which it is in contact.

4. Composite scaffold according to one of Claims 1 to 3, **characterized in that** the poly(lactic-co-glycolic acid) (PLGA) polymer has a lactic acid to glycolic acid ratio of 50/50.

5. Composite scaffold according to one of Claims 1 to 4, **characterized in that** the polyurethane (PU) is the product of a process, without any trace of catalyst, comprising:
- reacting 1,4-butanediol with ε-caprolactone having a molecular weight of approximately 1700 g/mol, to form a macrodiol having a molecular weight of between 1500 and 3000 g/mol;
- reacting the macrodiol obtained above with a diisocyanate to obtain a macrodiisocyanate;
- reacting the macrodiisocyanate obtained above with a chain extender, which chain extender is 1,4-butanediol.

6. Composite scaffold according to one of Claims 1 to 5, **characterized in that** it has a sufficient porosity which promotes the colonization of the composite scaffold by the fibrocartilage with which it is in contact and the growth of said fibrocartilage.

7. Composite scaffold according to one of Claims 1 to 6, **characterized in that** it is produced in a single form imitating the arched shape of the lateral or medial meniscus of the knee.

8. Composite scaffold according to one of Claims 1 to 7, for use as an implant in applications for knee meniscus replacement, and/or for treatment of osteoarthritis of the knee, in particular grade 3 and 4 osteoarthritis of the knee.

9. Implant for knee meniscus replacement, and/or for treatment of osteoarthritis of the knee, in particular grade 3 and 4 osteoarthritis of the knee, **characterized in that** it comprises a composite scaffold according to one of Claims 1 to 7.

10. Implant according to Claim 9, **characterized in that** it is produced in a single C-shaped form that can be used both for the lateral meniscus of the knee and for the medial meniscus of the knee.

11. Method for producing a composite scaffold according to one of Claims 1 to 7, comprising at least the steps consisting in:
- providing a porous polymeric structure as defined in one of Claims 1 to 7;
- applying collagen to the porous polymeric structure using a technique chosen from dipping, crosslinking and freeze-drying, or three-dimensional (3D) printing techniques so as to obtain the composite scaffold, the collagen being a recombinant human collagen type I or II produced from a plant, in particular from the tobacco plant.

12. Production method according to Claim 11, in which the collagen is applied to the porous polymeric structure by crosslinking and freeze-drying.

13. Production method according to Claim 11, in which the collagen is applied to the porous polymeric structure by three-dimensional (3D) printing.
